# EUROPEAN PATENT APPLICATION

(11) **EP 2 186 786 A2**
(43) Date of publication of application: **19.05.2010**
(21) Application number: 10151802.5
(22) Date of filing: 27.01.2010
(51) Int. Cl.: C07C 7/173, C07C 11/02

(54) **Process for the separation of olefins from paraffins**

(71) Applicant: Shell Internationale Research Maatschappij B.V., 2596 HR The Hague (NL)
(72) Inventor: de Boer, Eric Johannes Maria, 1031 HW, Amsterdam (NL); van der Heijden Harry, 1031 HW, Amsterdam (NL); van Zon, Arie, 1031 HW, Amsterdam (NL)

(57) **Abstract**

A process for the separation of internal olefins from a mixed feedstock comprising internal olefins and paraffins, wherein said process comprises:
(i) reacting said mixed feedstock with a trialkylaluminium compound in the presence of an isomerisation/displacement catalyst in order to cause isomerisation of the internal olefins and to displace alkyl group(s) from said trialkylaluminium compound to form an alkylaluminium compound wherein at least one of the alkyl groups bound to the aluminium is an alkyl group which has been derived from the isomerisation of said internal olefins; and
(ii) reacting the alkylaluminium compound formed in step (i) with one or more displacement olefins in the presence of an isomerisation/displacement catalyst in order to cause the displacement olefins to optionally isomerise and to displace alkyl group(s) from the alkylaluminium compound to form a trialkylaluminium compound and to liberate the 1-olefins derived from the internal olefins of the mixed feedstock, which 1-olefins are isomerised to form internal olefins.

## Description

### Field of the Invention

The present invention relates to a process for the separation of olefins, in particular internal olefins, from paraffins.

### Background of the Invention

Heavy internal olefins (e.g. C15-C30) are needed for the production of Internal Olefin Sulphonates (IOS) and/or their derivatives (according to EP 0446971 B1).

Lightly branched and linear heavy internal olefins are useful surfactant-precursors to be applied in enhanced oil recovery (EOR).

Furthermore, the internal olefin derived alcohols may serve this purpose after conversion to anionic surfactants by sulphation or sulphonation.

Appropriate internal higher olefin feeds can be obtained from the Shell Higher Olefin Process (SHOP) process and 1-olefin production processes as employed by Chevron-Phillips Chemical Company or INEOS (BP-AMOCO). However, as it is expected that large amounts of surfactants and hence, heavy internal olefins (e.g. C15-C30), are required for EOR, it is desirable if such olefins can also be obtained from other sources.

For example, the Fischer-Tropsch process utilises natural gas to produce paraffins. Flexibility would be increased if Fischer-Tropsch (Gas-To-Liquids (GTL)) paraffins could be efficiently converted to such olefins.

The dehydrogenation of higher paraffins leads to a mixture of olefins which mostly contain internal double bonds. As described in Encyclopedia of Chemical Processing and Design, 1982, Vol. 15, pp 266-284, a commercial process for the dehydrogenation of paraffins according is UOP's "PACOL" process. This process gives rise to the production of paraffin-diluted internal olefines (generally < 20 wt. % olefin content), as at higher conversions per pass dienes and aromatics are formed as by-products and moreover, coking phenomena are encountered in the "PACOL" dehydrogenation unit.

Separation/isolation of the internal olefins formed in this process is typically done for C10-C14 olefins by UOP's "OLEX" process, where the formed olefins are separated from the corresponding paraffins by selective adsorption. This separation enables paraffin recycle and eventually dehydrogenation of the recycled paraffin to extinction in the "PACOL" dehydrogenation unit.

However, the separation/isolation process by the "OLEX" technology is rather complex and hence, costly, in particular for the separation of C10-C14 internal olefins from its paraffin precursors.

For mixtures of hexenes, it has been shown in EP-A-0505834 and EP-A-0525760 that the exchange of propyl-groups of tripropylaluminium can be effected in the presence of an isomerisation/displacement catalyst, e.g. Ni(COD)₂. Hence, internal hexenes are transformed to trihexylaluminium, which in turn can be transformed into 1-hexene by action of propene and a non-isomerising displacement catalyst, e.g. cobalt naphthenate.

Said patent applications further disclose processes for preparing linear higher 1-olefins by successive transalkylation (isomerisation/displaceznent) reactions. In these publications, a linear, internal olefin having from 4 to 30 carbon atoms or a mixture of such olefins is reacted with trialkylaluminium compound in the presence of an isomerisation/displacement catalyst. This results in the formation of a trialkylaluminium compound in which at least one of the alkyl radicals is derived from the olefin used; this radical is present as a linear alkyl radical derived from the 1-olefin which has been formed by isomerisation. The trialkylaluminium compound is subsequently reacted with a 1-olefin in a displacement reaction in which the linear 1-olefin which was bound to the aluminium is liberated. This process allows internal olefins to be isomerised effectively and in good yields to produce terminal olefins.

US-A-5877378 discloses that 1-olefins can be extracted from a mixture of 1- and internal olefins by subjecting the mixture to tripropylaluminium and a non-isomerising displacement catalyst, e.g. cobalt naphthenate.

US 2004/0199035 A1 describes a process for the targeted preparation of long-chain 1-olefins having a narrow molecular weight distribution comprising the following steps:
i) introduction of a C4-C10-olefin fraction into an isomerising metathesis reaction,
ii) fractionation of the mixture obtained to give a) a C2-C3-olefin fraction, b) a fraction comprising olefins having the desired number of carbon atoms, c) a light fraction comprising olefins having a number of carbon atoms ranging from 4 to the integer below the number of carbon atoms of the desired fraction b) and d) a heavy fraction comprising olefins having a number of carbon atoms above that of the desired fraction b),
iii) recirculation of the light fraction c) and, if desired, the heavy fraction d) to the isomerising metathesis reaction i),
iv) reaction of the fraction b) with a trialkylaluminium compound in a transalkylation (alkyl group displacement) under isomerising conditions, in which an olefin corresponding to the alkyl radical is liberated and the olefins used add onto the aluminium with isomerisation and formation of corresponding alkylaluminium compounds,
v) reaction of the alkylaluminium compounds formed in iv) with an olefin to liberate the 1-olefins corresponding to the alkylaluminium compounds formed in step iv).

WO-A-2004/18388 describes a method for the production of 1-olefins from olefins which have internal double bonds, comprising: a) reacting an olefin with an internal double bond with a trialkylaluminium compound (A) in the presence of a catalyst in order to form a corresponding trialkylaluminium compound (B), wherein at least one of the alkyl groups which is connected to the aluminium is an alkyl group which is derived from 1-olefin, and b) separating the reaction mixture obtained in step a) from the used catalyst, and c) reacting the trialkylaluminium compound (B) formed in step a) with an olefin by displacing the desired alpha-olefin, whereby the reaction in step c) takes place thermally.

WO-A-2004/018389 discloses a method for the production of C4-C30 1-olefins from C4-C30-olefins which have internal double bonds, comprising: a) reacting an internal C4-C30-olefin with a trialkylaluminium compound in the presence of a nickel-free isomerisation/displacement catalyst containing at least one isomerisation active metal component and at least one displacement active metal component, by releasing an olefin which is derived from the initially bound alkyl radical, and forming a trialkylaluminium compound wherein at least one alkyl radical is derived from a C4-C30 olefin, b) reacting the trialkylaluminium compound formed in step a), which releases the desired C4-C30 1-olefins.

WO-A-2006/005762 discloses a process for the preparation of linear 1-olefins having 2n carbon atoms from linear 1-olefins having n carbon atoms comprising the steps of (a) dimerising a linear 1-olefin having n carbon atoms in the presence of a dimerisation catalyst to produce a linear internal olefin having 2n carbon atoms; (b)(i) reacting the linear internal olefin having 2n carbon atoms produced in step (a) with a trialkylaluminium compound in the presence of a catalytic amount of an isomerisation/displacement catalyst in order to cause isomerisation of the linear internal olefin and to displace alkyl group(s) from said trialkylaluminium compound to form an alkylaluminium compound wherein at least one of the alkyl groups bound to aluminium is a linear alkyl which has been derived from the isomerisation of said linear internal olefin; and (b) (ii) reacting said alkylaluminium compound with an alpha olefin optionally in the presence of a displacement catalyst so as to displace said linear alkyl from said alkylaluminium compound to form a linear 1-olefin having 2n carbon atoms.

However, the afore-mentioned prior art processes are targeted to the production of 1-olefins and are not concerned in any way with methods to separate internal olefins from dilute olefin/paraffin mixtures, in particular from dilute olefin/paraffin mixtures emerging from paraffin dehydrogenation by the "PACOL" process and/or from Fischer-Tropsch synthesis (e.g. a gas-to-liquids (GTL) intermediate product before hydrogenation, but after removal of the reactive by-products such as oxygenates).

Difficulties exist in separating mixtures of higher olefins and paraffins (> C₆) due to similarities in the boiling points and polarities thereof. These similarities render separation processes such as distillation, extraction and adsorption rather inefficient.

Thus, hitherto, it has not been suggested in the art that higher internal olefins (> C₆) can be isolated from dilute streams of paraffins of similar chain length, linearity, polarity and boiling point.

Hence, a cheap and straightforward technique for separating/isolating internal olefins from higher paraffin feedstocks (e.g. GTL's Heavy Detergent Feedstock, HDF, C14-C18 paraffins) and the dehydrogenated paraffins ex. "PACOL" process is required.

That is to say, it is highly desirable to find a process by which higher internal olefins (>C₆) can be isolated from a dilute olefin/paraffin mixture emerging, for example, from paraffin dehydrogenation by the "PACOL" process and/or from Fischer-Tropsch synthesis (e.g. a gas-to-liquids (GTL) intermediate product before hydrogenation, but after removal of the reactive by-products such as oxygenates).

In particular, it is highly desirable to develop a process for the separation of internal olefins with higher carbon numbers (> C₆) under high dilution in paraffins (e.g. to isolate internal olefins from a 10-20 wt. % mixture of internal olefins, in particular linear internal olefins, in the n-paraffins of similar carbon chain length).

### Summary of the Invention

According to the present invention there is provided a process for the separation of internal olefins from a mixed feedstock comprising internal olefins and paraffins, wherein said process comprises:
(i) reacting said mixed feedstock with a trialkylaluminium compound in the presence of an isomerisation/displacement catalyst in order to cause isomerisation of the internal olefins and to displace alkyl group(s) from said trialkylaluminium compound to form an alkylaluminium compound wherein at least one of the alkyl groups bound to the aluminium is an alkyl group which has been derived from the isomerisation of said internal olefins; and
(ii) reacting the alkylaluminium compound formed in step (i) with one or more displacement olefins in the presence of an isomerisation/displacement catalyst in order to cause the displacement olefins to optionally isomerise and to displace alkyl group(s) from the alkylaluminium compound to form a trialkylaluminium compound and to liberate 1-olefins derived from the internal olefins of the mixed feedstock, which 1-olefins are isomerised to form internal olefins.

### Detailed Description of the Invention

The starting mixed feedstock for use in the process of the present invention may be any feedstock comprising mixtures of internal olefins and paraffins.

Said mixed feedstock may comprise mixtures of linear and/or branched internal olefins and paraffins.

In a preferred embodiment of the present invention, the internal olefins in the mixed feedstock are preferably at least 90 wt. %, more preferably at least 95 wt. % linear internal olefins, with respect to the total weight of internal olefins in the mixed feedstock.

Said mixed feedstock is preferably a dilute olefin/paraffin mixture emerging, for example, from paraffin dehydrogenation by the "PACOL" process and/or from Fischer-Tropsch synthesis (e.g. a gas-to-liquids (GTL) intermediate product before hydrogenation, but after removal of the reactive by-products such as oxygenates).

Hence, in a preferred embodiment of the present invention, the mixed feedstock is derived from a Fischer-Tropsch process and/or the mixed feedstock is derived from the dehydrogenation of paraffins (or from an olefin/paraffin mixture, depleted of as much olefin as practicable).

Internal olefins and paraffins for separation by the process of the present invention typically have in the range of from 6 to 40 carbon atoms, preferably in the range of from 8 to 32 carbon atoms, more preferably in the range of from 10 to 30 carbon atoms, even more preferably in the range of from 14 to 24 carbon atoms and most preferably in the range of from 14 to 18 carbon atoms.

The mixed feedstock preferably comprises in the range of from 1 to 40 wt. %, more preferably in the range of from 2 to 30 wt. % and most preferably in the range of from 5 to 25 wt. %, of internal olefins, with respect to the total weight of said feedstock.

Thus, in a preferred embodiment of the present invention there is provided a process for the separation of internal olefins having in the range of from 6 to 40 carbon atoms, preferably in the range of from 8 to 32 carbon atoms, more preferably in the range of from 10 to 30 carbon atoms, even more preferably in the range of from 14 to 24 carbon atoms and most preferably in the range of from 14 to 18 carbon atoms from a mixed feedstock comprising internal olefins and paraffins having in the range of from 6 to 40 carbon atoms, preferably in the range of from 8 to 32 carbon atoms, more preferably in the range of from 10 to 30 carbon atoms, even more preferably in the range of from 14 to 24 carbon atoms and most preferably in the range of from 14 to 18 carbon atoms, wherein said process comprises:
(i) reacting said mixed feedstock with a trialkylaluminium compound in the presence of an isomerisation/displacement catalyst in order to cause isomerisation of the internal olefins and to displace alkyl group(s) from said trialkylaluminium compound to form an alkylaluminium compound wherein at least one of the alkyl groups bound to the aluminium is an alkyl group which has been derived from the isomerisation of said internal olefins; and
(ii) reacting the alkylaluminium compound formed in step (i) with one or more displacement olefins in the presence of an isomerisation/displacement catalyst in order to cause the displacement olefins to optionally isomerise and to displace alkyl group(s) from the alkylaluminium compound to form a trialkylaluminium compound and to liberate the 1-olefins derived from the internal olefins of the mixed feedstock, which 1-olefins are isomerised to form internal olefins having in the range of from 6 to 40 carbon atoms, preferably in the range of from 8 to 32 carbon atoms, more preferably in the range of from 10 to 30 carbon atoms, even more preferably in the range of from 14 to 24 carbon atoms and most preferably in the range of from 14 to 18 carbon atoms.

In step (i) of the process of the present invention the mixed feed is reacted with a trialkylaluminium compound in the presence of a catalytic amount of an isomerisation/displacement catalyst in order to cause isomerisation of the internal olefins and to displace alkyl group(s) from said trialkylaluminium compound to form an alkylaluminium compound wherein at least one of the alkyl groups bound to the aluminium is an alkyl group which has been derived from the isomerisation of said internal olefins and a 1-olefin corresponding to the displaced alkyl group(s) from said trialkylaluminium compound. Said displaced 1-olefin may undergo subsequent isomerisation in the presence of the isomerisation/displacement catalyst in step (i) to form one or more internal olefins.

While not wishing to be bound by theory, it is believed that the reaction in step (i) of an internal olefin with a trialkylaluminium compound in the presence of an isomerisation/displacement catalyst causes isomerisation of the internal olefin to form at least some 1-olefin, which 1-olefin displaces alkyl groups from said trialkylaluminium compound to form an alkylaluminium compound wherein at least one of the alkyl groups bound to aluminium is an alkyl group which has been derived from said 1-olefin.

In step (i), the internal olefin is preferably reacted with the trialkylaluminium compound in a molar ratio in the range of from 1:1 to 50:1, preferably in the range of from 3:1 to 10:1.

Preferred catalysts for use in step (i) and/or step (ii) are those catalysts which catalyse both isomerisation and displacement, hence the use of the term "isomerisation/displacement catalyst".

The isomerisation/displacement catalyst for use in step (i) and/or step (ii) can be any catalyst suitable for isomerising an internal olefinic double bond, but is preferably a nickel based isomerisation/displacement catalyst, such as those disclosed in EP-A-0505834 and EP-A-0525760.

The isomerisation/displacement catalyst used herein is preferably selected from nickel (II) salts, nickel (II) carboxylates, nickel (II) acetonates and nickel (0) complexes and mixtures thereof. Said isomerisation/displacement catalyst may be stabilised by means of a ligand, such as a trivalent phosphorus ligand.

Examples of nickel (II) salts include nickel halides e.g. nickel (II) chloride, nickel (II) bromide, nickel (II) iodide, and their hydrates. Also useful are nickel (II) oxide and nickel (II) hydroxide.

Examples of suitable nickel salts include carboxylates, carbamates, alkoxides, thiolates, catecholates, oxalates, thiocarboxylates, tropolates, phosphinates, acetylacetonates, iminoacetylacetonates, bis-iminoacetylacetonates, the solubility of which can be tuned by an appropriate choice of substituents, as well known to those skilled in the art.

Preferred metal salts for use herein are the optionally substituted acetylacetonates, or x, (x+2)-alkanedionates, where x is an integer e.g. 2 to 6 such as 2,4-alkanedionates and 3,5-alkanedionates. When the acetylacetonates are substituted, preferred substituents are C₁-C₆ alkyl groups, especially methyl. Examples of suitable acetylacetonates include 2,4-pentanedionates, 2,2,6,6-tetramethyl-3,5-heptanedionates. Other examples are aryl substituted y, (y+2)-alkanedionates such as 1-phenyl-1,3-butanedionates and 1,3-diphenyl-1,3-propanedionates. Preferred acetylacetonates for use herein are the 2,4-pentanedionates.

Examples of nickel (II) carboxylates include nickel acetate, nickel 2-ethylhexanoate, nickel octanoate and nickel naphthenate.

An example of a nickel acetonate which may be conveniently used is nickel (II) acetylacetonate.

Examples of Ni(0) complex catalysts include Ni(CO)₄ and nickel (0) olefin complexes such as nickel bis-1,5-cyclooctadiene (Ni(COD)₂), Ni(C₂H₄)₃, Ni(norbornene)₃ and nickel cyclododecatriene.

A particularly preferred isomerisation/displacement catalyst for use in step (i) and/or step (ii) of the process of the present invention is nickel bis-1,5-cyclooctadiene (Ni(COD)₂).

The isomerisation/displacement catalyst used in step (ii) of the process of the present invention is preferably identical to the isomerisation/displacement catalyst used in step (i) of the present invention.

However, as used herein, the term "isomerisation/displacement catalyst" also allows for separate isomerisation catalysts and displacement catalysts to be used for the isomerisation/displacement reactions in steps (i) and/or (ii), provided that said catalysts do not interfere with each other.

Examples of isomerisation catalysts that may be conveniently used include sodium, potassium and sodium-potassium alloys on aluminium oxide.

Examples of displacement catalysts that may be conveniently used include, for example, colloidal Ni, Pt, Co, nickel acetylacetonate, cobalt carboxylates, e.g. cobalt naphthenate or cobalt acetate, nickel carboxylates, e.g. nickel naphthenate and the like.

In the event that separate isomerisation catalysts and displacement catalysts are used for the isomerisation/displacement reactions in steps (i) and (ii), then it is preferred that the combination of isomerisation catalysts and displacement catalysts used in each step is identical.

The trialkylaluminium compounds suitable for use in step (i) of the process of the present invention are known to those skilled in the art. Preferably, the alkyl groups of the trialkylaluminium compounds contain fewer carbons than the predominant carbon number of the internal olefins to be separated.

Trialkylaluminium compounds which may be conveniently used in step (i) are those which contain alkyl groups having in the range of from 2 to 24 carbon atoms, preferably in the range of from 2 to 18 carbon atoms, more preferably in the range of from 2 to 12 carbon atoms.

Examples of trialkylaluminium compounds which may be used include triethylaluminium, tri-n-propylaluminium, tri-n-butylaluminium, tri-isobutylaluminium, tri-n-hexylaluminium, tri-n-octylaluminium, tri-n-decylaluminium, tri-n-dodecylaluminium, tri-n-tetradecylaluminium, tri-n-hexadecylaluminium, tri-n-octadecylaluminium and mixtures thereof. A particularly preferred trialkylaluminium compound for use in step (i) is tri-n-octylaluminium.

In step (i) of the process of the present invention, the isomerisation/displacement catalyst can be added to a mixture of the trialkylaluminium compound and the mixed feedstock comprising internal olefins and paraffins.

Alternatively, the isomerisation/displacement catalyst can be first mixed with the mixed feedstock comprising internal olefins and paraffins and this mixture can then be added to the trialkylaluminium compound.

Both isomerisation and displacement can be simultaneously carried out in the same vessel. Alternatively, the isomerisation reaction can be initiated in a first reactor and then fed to a second reactor containing the trialkylaluminium compound. The reaction can be carried out in a batch or continuous manner.

Step (i) is carried out in the presence of a catalytic amount of a isomerisation/displacement catalyst. The isomerisation/displacement catalyst is preferably present in the reaction mixture of step (i) in an amount in the range of from 0.1 to 1000 mg of metal per kg of total solution, more preferably in the range of from 3 to 300 mg of metal per kg of total solution and most preferably in the range of from 5 to 100 mg of metal per kg of total solution.

In the event that separate isomerisation catalysts and displacement catalysts are used in step (i), then displacement catalyst is preferably present in an amount in the range of from 0.1 to 1000 mg of metal per kg of total solution, more preferably in the range of from 3 to 300 mg of metal per kg of total solution and most preferably in the range of from 5 to 100 mg of metal per kg of total solution.

In order to favour the replacement of the alkyl groups by the isomerised internal olefins in step (i), the displaced alkyl groups in the form of their corresponding 1-olefins or internal olefins can be removed as vapour from the reaction mixture. Optionally, said displaced 1-olefins or internal olefins from step (i) can be used as displacement olefins in the recovery of isomerised internal olefins by back-displacement in step (ii) of the process of the present invention. That is to say, the displacement olefins used in step (ii) may be derived from the displaced alkyl group(s) from the trialkylaluminium compound of step (i).

Unreacted internal olefins from the mixed feedstock can be separated from the reaction mixture prior to step (ii) using conventional methods such as by distillation or vacuum stripping and can be recycled to the isomerisation/displacement reaction of step (i).

Typical reaction temperatures for step (i) of the process of the present invention are in the range of from -20 to 200 °C, preferably in the range of from 30 to 100 °C.

Typical reaction pressures for steps (i) and/or (ii) of the process of the present invention are in range of from 0 to 10 MPa (0 to 100 bar(a)), preferably in the range of from 0.01 to 5 MPa (0.1 to 50 bar(a)), more preferably in the range of from 0.01 to 0.3 MPa (0.1 to 3 bar(a)).

Typical reaction times in step (i) of the process of the present invention usually range from 0.1 to 2 hours.

Solvents are not necessary for the isomerisation/displacement reactions of steps (i) and/or (ii) of the process of the present invention but can be used if desirable. Suitable solvents include inert aliphatic and aromatic hydrocarbons.

It is sometimes advantageous, especially when using a reactor in which distillation is also taking place, to include an inert diluent such as isoheptane, heptane, octane, or isooctane in the feed.

Thus, in a preferred embodiment of the process of the present invention, step (i) is performed in the presence of a solvent or diluent at least some of which remains at the end of the reaction of step (i) along with the product alkylaluminium compound, which alkylaluminium compound has at least one alkyl group bound to the aluminium that is an alkyl group which has been derived from the isomerisation of said internal olefins.

In a preferred embodiment of the present invention, the 1- and internal olefins displaced from the trialkylaluminium compound of step (i) are separated prior to step (ii).

In a particularly preferred embodiment, the reaction of step (i) can be encouraged to go to completion by distillation of the olefins displaced from the trialkylaluminium compound in step (i) in order to leave a solution or suspension of isomerisation/displacement catalyst with the product alkylaluminium compound, which alkylaluminium compound has at least one alkyl group bound to the aluminium that is an alkyl group which has been derived from the isomerisation of said internal olefins.

This solution or suspension can be used as such in the step (ii) of the process of the present invention without purification, or may be treated to separate the catalyst first especially if it is insoluble, before the product alkylaluminium compound is used in step (ii).

Step (ii) of the process of the present invention is a so-called back-displacement reaction wherein the alkyl groups from the isomerised internal olefins are back-displaced from the alkylaluminium compound formed in the isomerisation/displacement reaction of step (i), by reaction with one or more displacement olefins in the presence of an isomerisation/displacement catalyst in order to cause the displacement olefins to optionally isomerise and to displace alkyl group(s) from the alkylaluminium compound to form a trialkylaluminium compound and to liberate the 1-olefins derived from the internal olefins of the mixed feedstock, which 1-olefins are isomerised to form internal olefins.

In step (ii) of the process of the present invention, the displacement olefins may be internal or 1-olefins. It will be appreciated that when the displacement olefins are 1-olefins, then they will react directly will react alkylaluminium compound formed in the isomerisation/displacement reaction of step (i), whereas when the displacement olefins are internal olefins they will undergo isomerisation to 1-olefins in the presence of the isomerisation/displacement catalyst in step (ii) prior to reaction with the alkylaluminium compound formed in the isomerisation/displacement reaction of step (i).

Similarly, it will be appreciated that the displaced olefins in steps (i) and (ii) are primarily, that is to say, preferably more than 95 wt. %, 1-olefins which may be subsequently isomerised to internal olefins in the present of the isomerisation/displacement catalyst that is present in steps (i) and (ii).

In one embodiment of the present invention, the olefin reactants, that is to say, the displacement olefins used for the back-displacement reaction in step (ii), are derived from the displaced alkyl group(s) of the trialkylaluminium compounds of step (i). That is to say, displaced olefins recovered from the isomerisation/displacement reaction of step (i) as described above can be used as said displacement olefins. The trialkylaluminium compound regenerated in step (ii) can then be recycled back to the isomerisation/displacement reaction of step (i).

Alternatively, in another embodiment of the present invention, different olefins can be used as the displacement olefins for the back-displacement reaction of step (ii).

One or more displacement olefins, that is to say internal and/or 1-olefins, having preferably in the range of from 2 to 18 carbon atoms, more preferably in the range of from 3 to 16 carbon atoms and most preferably in the range of from 4 to 14 carbon atoms, may be conveniently used in back-displacement reaction of step (ii).

For example, said displacement olefins may be conveniently selected from internal hexene, internal heptene, internal octene, internal nonene, internal decene, internal undecene, internal dodecene, 1-hexene, 1-octene, 1-decene and 1-dodecene.

Particularly preferred displacement olefins for use in back-displacement reaction of step (ii) are internal octene, internal decene, internal dodecene, 1-octene, 1-decene and 1-dodecene.

The amount of displacement olefins used in the back-displacement reaction of step (ii) is preferably in a stoichiometric excess over the amount required to replace all of the alkyl group(s) in the alkylaluminium compound formed in step (i), preferably at least a 200 % excess, more preferably an excess in the range of from 200 to 3000 %.

The displacement olefins used for the back-displacement reaction in step (ii) may be fresh olefins or a mixture thereof with displaced 1-olefins and/or internal olefins from step (i).

The displacement olefins in step (ii) may comprise an equilibrium or non-equilibrium mixture of 1-olefin and at least one internal isomer thereof having the same carbon skeleton; examples of such mixtures are mixtures of 1-octene and a *cis*/*trans* mixture of 2-octene, 3-octene and 4-octene, preferably those produced as a result of isomerisation.

The displacement olefins used for displacement in step (ii) may also comprise an excess of olefin unreacted in step (ii) and recycled.

Thus, in a preferred embodiment of the present invention, the displacement olefins used in step (ii) of the process of the present invention are a mixture of olefins, in particular 1-olefins, displaced from step (i) and olefins, in particular 1-olefins, unreacted from step (ii).

As mentioned above, when the displacement olefins comprise 1-olefins and are isomerisable under the reaction conditions of step (ii), the unreacted olefin stream from step (ii) may also comprise isomerised olefins such as internal olefins, e.g. 2-octene when 1-octene is the displacement olefin.

Step (ii) is carried out in the presence of a catalytic amount of a isomerisation/displacement catalyst. Said catalyst may be preferably used in an amount in the range of from 0.1 to 1000 mg of metal per kg of total solution, more preferably in the range of from 3 to 300 mg of metal per kg of total solution and most preferably in the range of from 5 to 100 mg of metal per kg of total solution.

In the event that separate isomerisation catalysts and displacement catalysts are used in step (ii), then the displacement catalyst is preferably present in an amount in the range of from 0.1 to 1000 mg of metal per kg of total solution, more preferably in the range of from 3 to 300 mg of metal per kg of total solution and most preferably in the range of from 5 to 100 mg of metal per kg of total solution.

Isomerisation/displacement catalysts that may be conveniently used in step (ii) are those described above for use in step (i).

As per step (i), examples of said catalysts include nickel complexes such as nickel acetylacetonate, nickel carboxylates such as nickel naphthenate and nickel acetate.

As described above, separate isomerisation catalysts and displacement catalysts may also be used for the isomerisation/displacement reactions in step (ii), provided that said catalysts do not interfere with each other.

The back-displacement reaction of step (ii) may be generally carried out at a reaction temperature in the range of from -20 to 200 °C, preferably in the range of from 0 to 100 °C and more preferably in the range of from 0 to 50 °C.

Step (ii) may be performed at a temperature about the same as step (i), e.g. plus or minus 10 °C. However, preferably step (ii) is performed at a lower temperature such as 30 to 80 °C lower than step (i).

As mentioned above, typical reaction pressures for step (ii) of the process of the present invention are in range of from 0 to 10 MPa (0 to 100 bar(a)), preferably in the range of from 0.01 to 5 MPa (0.1 to 50 bar(a)), more preferably in the range of from 0.01 to 0.3 MPa (0.1 to 3 bar(a)).

Step (ii) is typically carried out over a period of from 30 seconds to 1 hour (at 25 °C), preferably from 1 minute to 20 minutes.

Step (ii) of the process of the present invention liberates 1-olefines from the alkylaluminium compound formed in step (i) and also produces a trialkylaluminium compound based on the displacement olefins added to step (ii). Said 1-olefins are derived from the internal olefins of the mixed feedstock and are isomerised to form internal olefins.

The reaction product mixture from step (ii) also usually contains isomerisation/displacement catalyst and often residual unreacted displacement olefins. It may also contain some residual isomerisation/displacement catalyst from step (i), which catalyst may or may not be the same as the isomerisation/displacement catalyst used in step (ii).

Thus, at the end of step (ii) the product reaction mixture generally contains the product isomerised internal olefins, possibly some 1-olefins formed in step (ii) which have not undergone isomerisation, unreacted displacement olefins, trialkylaluminium compound and possibly, but preferably, solvent/diluent, and/or isomerisation/displacement catalyst from step (ii) and/or residual displacement/isomerisation catalyst which had been carried over from step (i).

Distillation of the product reaction mixture of step (ii) can separate the product isomerised internal olefins, any 1-olefins formed in step (ii) which have not undergone isomerisation and any residual unreacted displacement olefins and optionally at least some of the solvent/diluent from the remainder.

In circumstances wherein the displaced olefins recovered from the isomerisation/displacement reaction of step (i) are used as the olefin reactants in step (ii), after removal from the remainder of any residual isomerisation/displacement catalyst(s) from steps (i) and (ii), e.g. by filtration or distillation, the regenerated trialkylaluminium compound can then be recycled to step (i).

The residual isomerisation/displacement catalyst from step (i) may have reduced isomerisation activity but sufficient activity for step (ii) and so may be used in step (ii). However, in a preferred embodiment of the present invention, an additional quantity of fresh isomerisation/displacement catalyst will be used in step (ii), either alone, or in addition to residual isomerisation/displacement catalyst from step (i).

In a preferred embodiment of the present invention, the isomerisation/displacement catalyst used in step (ii) and/or residual isomerisation/displacement catalyst remaining from step (i) are deactivated or removed, e.g. by adsorption and/or by filtration or distillation, to be optionally reused again in step (i), prior to isolating the product isomerised internal olefins from the reaction mixture of step (ii).

Thus, in a particularly preferred embodiment of the present invention, the displacement/isomerisation catalyst and trialkylaluminium compound may be used in step (i) and then for step (ii) and then back in step (i), the nature of the trialkylaluminium compound present oscillating between the two steps. The steps (i) and (ii) may be performed in the same reactor or two reactors in series. Unreacted displacement olefins from step (ii) can be recycled for reuse in step (ii).

Thus, the process of the present invention provides a convenient method for separating internal olefins from a mixed feed comprising internal olefins and paraffins.

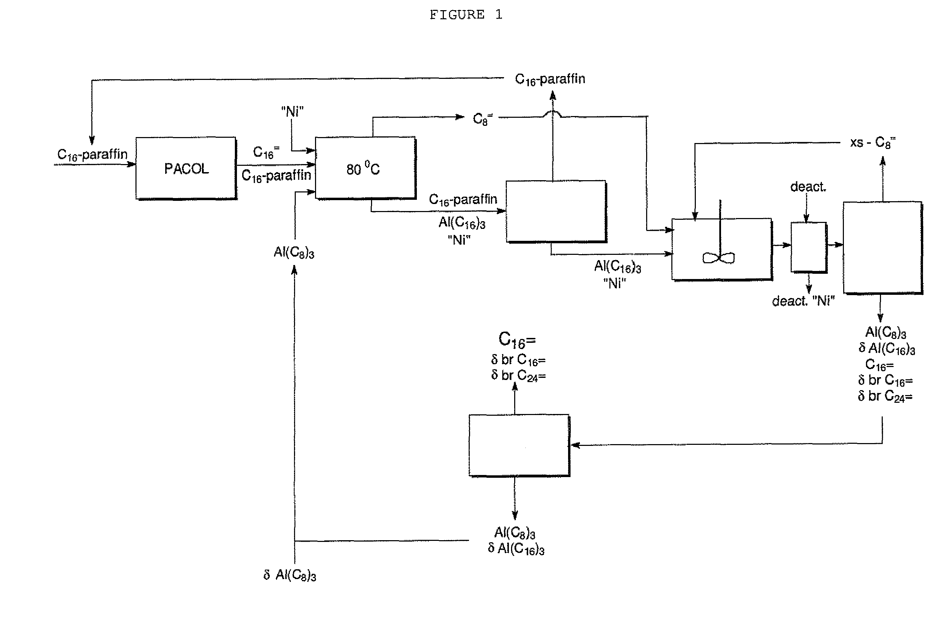

As outlined in Figure 1, in a preferred embodiment of the process of the present invention, linear or lightly branched internal hexadecene(s), prepared by dehydrogenation in the "PACOL" process at 20 wt. % concentration from largely linear hexadecane from the Fischer-Tropsch process may be separated from this diluted mixture in hexadecane after isomerisation/displacement reactions with trioctylaluminium.

Thus, in the presence of an isomerisation/displacement catalyst, such as Ni(COD)₂, Ni 2-ethylhexanoate or Ni naphthenate (a zerovalent-divalent nickel containing compound, indicated henceforth as "Ni" catalyst), a mixture of trihexadecylaluminium, hexadecane and 1-octene and internal octenes are initially formed. The isomerisation/displacement reaction is driven to completion by boiling off the octenes (see Figure 1).

The internal octenes, which are used in excess in step (ii) for their transformation into tri-n-octylaluminium can eventually be isolated from step (i) as pure 1-octene. This transformation of low-value internal octenes into high-value 1-olefin is an additional advantage of the process of the present invention. The purity of 1-octene formed in step (i) depends on the residence time in the displacement/distillation unit or rather its contact time with the "Ni" catalyst in solution in step (i). In this regard, it is of note that Examples 14 to 17 herein show that in the presence of trihexylaluminium the rate of "Ni" catalysed double bond isomerisation of trans-2-octene at 77 °C is rather low (26-35 % double bond isomerisation after 2 hours depending on the character of the "Ni" catalyst, the Al(hexyl)₃/Ni ratios and contents).

Trihexadecylaluminium can further be separated from hexadecane by distillation.

In step (ii), upon treatment of the heavy residue, trihexadecylaluminium, with an excess of octenes in the presence of an isomerisation/displacement "Ni" catalyst, as described above, largely linear internal hexadecenes (and a small amount of branched C₁₆-internal olefins (IO's) and an even smaller amount branched C₂₄-IO's, emerging from 1-octene insertion into the 1-octylaluminium bond or into the 1-hexadecylaluminium bond during the isomerising back-displacement reaction of step (ii)) and trioctylaluminium are formed.

If, however, the excess of olefins is removed in the presence of the isomerisation/displacement catalyst, the displacement equilibrium between 1-hexadecyl and 1-octyl groups on aluminium would be reversed. This is due to the fact that the ratio of 1-hexadecyl and 1-octyl groups on aluminium is dependent on the molar ratio of the respective olefins in the solution in the presence of an active isomerisation/displacement catalyst. To evade this reverse reaction upon boiling off the octenes the "Ni" isomerisation/displacement catalyst is deactivated or removed before isolation of the constituents by subsequent distillations.

As mentioned above, the displacement olefins (i.e. octenes) may form small amounts of branched C₁₆ and C₂₄ in side-reactions, i.e. the insertion of 1-octene into 1-octyl- and 1-hexadecylaluminium, which are important constituents for the final olefinic product, and also reform tri-n-octylaluminium. The latter product is recycled to the dilute hexadecene stream of step (i).

Hence, by application of only one single isomerisation/displacement catalyst, a simpler and more robust process is obtained compared to WO-A-2006/005762.

The process of the present invention may be performed in a batch, semi batch or preferably continuous manner.

In a preferred embodiment of the present invention, (a) 1- and internal olefins displaced from the trialkylaluminium compound of step (i) are separated prior to step (ii) and/or (b) the isomerisation/displacement catalyst remaining from step (i) are deactivated prior to isolating said isomerised internal olefins from the reaction mixture of step (ii).

In a particularly preferred embodiment of the present invention, there is provided a process for the separation of internal olefins from a mixed feedstock comprising internal olefins and paraffins, wherein said process comprises:
(i) reacting said mixed feedstock with a trialkylaluminium compound in the presence of an isomerisation/displacement catalyst in order to cause isomerisation of the internal olefins and to displace alkyl group(s) from said trialkylaluminium compound to form an alkylaluminium compound wherein at least one of the alkyl groups bound to the aluminium is an alkyl group which has been derived from the isomerisation of said internal olefins;
(ii) separating from the reaction mixture of step (i), olefins liberated from said trialkylaluminium compound and the paraffins;
(iii) treating the heavy residue of step (ii) with one or more displacement olefins in the presence of an isomerisation/displacement catalyst in order to cause the displacement olefins to optionally isomerise and form a trialkylaluminium compound and to liberate 1-olefins derived from the internal olefins of the mixed feedstock from the alkylaluminium compound, which 1-olefines are isomerised to form internal olefins;
(iv) deactivating or removing said isomerisation/displacement catalyst; and
(v) isolating from the reaction mixture of step (iv) said isomerised internal olefins.

In the afore-mentioned particularly preferred embodiment, it will be appreciated that step (i) corresponds to step (i) as hereinbefore described and step (iii) corresponds to step (ii) as hereinbefore described.

The present invention is described below with reference to the following Examples, which are not intended to limit the scope of the invention in any way.

### EXAMPLES

### General Procedures and Characterisation

All chemicals used in preparations were purchased from Aldrich and used without further purification unless mentioned otherwise.

All the operations with the catalyst systems were carried out under nitrogen atmosphere. All solvents used were dried using standard procedures.

Anhydrous o-xylene (> 97 % purity) was stored over Na-wire and 4Å molecular sieves (final water content of about 3 ppm).

The products obtained were characterised by Gas Chromatography (GC) in order to evaluate yield of the various compounds using a HP 5890 series II apparatus. Generally the following chromatographic conditions were applied:

Column: HP-1 (cross-linked methyl siloxane), film thickness = 0.25 µm, internal diameter = 0.25 mm, length 60 m (by Hewlett Packard); injection temperature: 325 °C; detection temperature: 325 °C; initial temperature: 40 °C for 10 minutes; temperature programme rate: 10.0 °C/minute; final temperature: 325 °C for 41.5 minutes; internal standard: o-xylene or hexadecane.

The NMR data was obtained at room temperature with a Varian 300 MHz or 400 MHz apparatus.

To improve the accuracy of analysis, Examples 1-12 are experiments using model-mixtures of internal olefins and n-paraffins in order to establish preferred conditions for the separation of olefins from paraffins.

These conditions have subsequently been applied in Example 13 to a mixture representative of a "PACOL" C₈-product stream.

### Examples 1-12

In a pressure vessel, the amount of olefin (mixture), the amount of paraffin, the amount of Al(n-C₆H₁₃)₃ (94.9% purity) and the amount of solution of Ni(COD)₂ (0.75 mg of Ni(COD)₂/g of heptane) to attain the Ni-content on total intake, as described in Table 1, were weighed in under inert conditions together with 64 mg n-hexadecane (internal standard).

The reaction vessel was generally heated for 1 hour at 80 °C, subsequently cooled to room temperature and a 0.3 g sample was taken.

The sample was diluted with pentane, quenched with 0.5 N sulphuric acid and vigorously shaken. The organic layer was analysed by gas chromatography (GC).

In a typical example of an isomerisation/displacement experiment (Example 8), 3.57 mmol *trans*-2-octene in 1.613 g *n*-tetradecane in contact with 0.38 mmol Al(n-C₆H₁₃)₃ was used.

GC showed the presence of a C₆ fraction consisting of *n*-hexane, 1-hexene and internal hexenes, of a C₈ fraction consisting of *n*-octane, 1-octene and internal octenes, and finally pentane, heptane, *n*-tetradecane and *n*-hexadecane.

On the basis of the ratio of hexane and hexenes, the exchange of hexyl groups on aluminium by octenes was calculated. Since the initial molar ratio of hexyl groups of trihexylaluminium and octene was also known, a conversion relative to the theoretical equilibrium ratio of octyl over hexyl groups attached to aluminium (theoretical equilibrium) was calculated (as 83.3% for Example 8), i.e. the theoretical equilibrium ratio of octyl over hexyl groups attached to aluminium is defined as the molar ratio of all the octene isomers over the hexyl groups on aluminium, initially present.

These conversions relative to the theoretical equilibrium, which are given in Table 1, are a measure of the isomerisation/displacement efficiency.

**Table 1**

| Example | Trihexyl-aluminium (THA) (mmol) | Olefin (mmol) | Paraffin (g) | Olefin (Olefin+Paraffin) (wt.%) | Ni (COD) _{2'} as Ni on total solution, mg/kg; (%mol/mol on THA) | Time (h) | Conversion, relative to theoretical equilibrium; by C₆-analysis (%) |
|---|---|---|---|---|---|---|---|
| 1 | 0.54 | Internal tetradecenes (1.60) | Octane (2.811) | 10 | 87 (1.0) | 1 | 23 |
| 2 | 0.54 | Internal tetradecenes (1.60) | Octane (2.811) | 10 | 87 (1.0) | 2 | 23 |
| 3 | 0.54 | Internal tetradecenes (1.60) | Octane (2.811) | 10 | 87 (1.0) | 20 | 28 |
| 4 | 0.37 | Trans-2-octene (1.12) | Tetra-decane (0.493) | 20 | 92 (0.3) | 1 | 53 |
| 5 | 0.39 | Trans-2-octene (1.13) | Tetra-decane (0.494) | 20 | 288 (1.1) | 1 | 65 |
| 6 | 0.39 | Trans-2-octene (1.06) | Tetra-decane (0.490) | 20 | 28 (0.1) | 1 | 44 |
| 7 | 0.39 | Trans-2-octene (0.69) | Tetra-decane (0.299) | 21 | 27 (0.07) | 1 | 35 |
| 8 | 0.38 | Trans-2-octene (3.57) | Tetra-decane (1.613) | 20 | 28 (0.3) | 1 | 83 |
| 9 | 0.38 | Trans-2-octene (3.61) | Tetra-decane (0.140) | 74 | 27 (0.09) | 1 | 86 |
| 10 | 0.38 | Trans-2-octene (3.60) | - | 100 | 30 (0.08) | 1 | 85 |
| 11 | 0.087 | Trans-2-octene (0.84) | Heptane (3.803) | 10 | 30 (0.6) | 1 | 68 |
| 12 | 0.042 | Trans-2-octene (0.41) | Heptane (3.997) | 5 | 30 (1.2) | 1 | 71 |

In Examples 1-3 it is shown that a 10 wt. % solution of linear internal tetradecenes in paraffin reacts with trihexylaluminium (THA at about equivalent amounts of olefin and hexyl groups) in the presence of 87 ppm (wt/wt Ni on total solution) of Ni(COD)₂ to a conversion of 23-28 % of the theoretical equilibrium.

It is clear that upon increase of the duration of the experiment the conversion hardly changes (Examples 1-3).
Hence, after one hour the reaction is close to completion.

If the mixture of linear internal tetradecenes is replaced by *trans*-2-octene and the olefin concentration increases to 20 wt. %, i.e. more than two-fold increase of the double bond concentration, the conversion increases to 53 % of the theoretical equilibrium (Example 4) .

Upon increase or decrease of the amount of Ni(COD)₂, the conversion increases or decreases gradually (Examples 5 and 6).

Increase of the concentration of Ni(COD)₂, however, renders the process economically less attractive (Example 5).

Increase of the amount of trihexylaluminium to a two-fold excess of hexyl-groups over olefin at a low level of Ni(COD)₂ results in a decrease of the conversion to from 44 to 35 % of the equilibrium value (Examples 6 and 7).

Upon increase of the amount of internal olefin over trihexylaluminium (Example 8), the conversion increases to 83 % of the theoretical equilibrium, which is almost as high as the conversion level observed for the similar experiments with less-diluted (74 wt. %) and the undiluted *trans*-2-octene (Examples 9 and 10, respectively).

Upon dilution of the olefin below 20 wt. %, but keeping the molar ratio of olefin and trihexylaluminium and the Ni concentration on total solution the same, results in a somewhat lower conversion (compare Examples 8, 11 and 12).

### Example 13

In a pressure vessel, 941 mg (8.40 mmol) of a mixture of *cis+trans*-2-octene, *cis+trans*-3-octene and *cis+trans-*4-octene with an isomer ratio of 39:36:25 and 3.65 g of *n*-octane, 156 mg (0.55 mmol) of Al(*n*-C₆H₁₃)₃ (94.9% purity) and 199 mg of a solution of Ni(COD)₂ in heptane (3 mg of Ni(COD)₂/g of heptane amounting to about 30 ppm Ni on total intake) were weighed in under inert conditions together with 246 mg o-xylene and 53 mg *n-*hexadecane (internal standards).

The reaction vessel was heated for 1 hour at 80 °C, subsequently cooled to room temperature.

After taking a 193 mg sample the volatiles of the thus obtained reaction mixture were distilled off at room temperature under vacuum.

After distillation 207 mg of residue remained, while 4.73 g volatiles were collected as distillate. GC analysis of the distillate did not show the presence of any *n*-hexadecane.

A sample of 18 mg of the residue was diluted in pentane, quenched with diluted sulphuric acid, and the organic products analyzed by gas chromatography.

GC showed the presence of a C₆ fraction consisting of *n*-hexane, of a C₈ fraction consisting of *n*-octane, and of *n*-hexadecane (internal standard), pentane and only a trace of o-xylene (internal standard). On the basis of the C₆ and C₈ data the formula of the original Al compound can be calculated as Al(C₆H₁₃)₁.₂₇(C₈H₁₇)_{1.73}.

The residue obtained from the experiment described above, 189 mg, was reacted with 2.57 g (30.6 mmol) of 1-hexene in the presence of 24 mg of a solution of Co naphthenate in nonane (1.2 mg of Co/g of nonane amounting to 10 ppm Co on total intake).

After stirring the reaction mixture 15 minutes at room temperature, a sample was taken, quenched with acid, and the organic components analysed by means of gas chromatography.

GC showed the presence of a C₈ fraction consisting of 91.3% 1-octene, 5.9% (*n*-octane + octene isomer) and 2.8% internal octenes. From the 5.9% (*n*-octane + octene isomer) fraction at the most 0.6% is attributable to the octene isomer. The total selectivity to linear octenes defined as the selectivity to 1-octene + selectivity to *n*-octane is 96.6%. GC analyses of a sample taken after 30 minutes of reaction showed the same results.

This example shows that a dilute mixture of internal octenes can be converted to linear 1-octene and separated from the octane diluent by using the process of the present invention with high yield and high selectivity.

When, instead of the non-isomerising displacement catalyst, Co naphthenate, an isomerisation/displacement catalyst, Ni 2-ethylhexanoate (ca. 200 ppm Ni on total intake), was employed in the above-described back-displacement reaction, using a large excess of internal hexenes (hexenes : Al ratio = 50 mol/mol) at room temperature the following was observed.

After stirring the reaction mixture for 4 hours at room temperature a sample was taken, quenched with acid, and the organic components analysed by means of gas chromatography.

GC showed the presence of a C₈ fraction consisting mainly of internal octenes with only ~1% 1-octene and some 5% *n*-octane left.

It is apparent that the conditions established in Example 8 have successfully been applied in Example 13 to a mixture representative of a "PACOL" C₈-product stream.

This Example surprisingly shows that a dilute mixture of internal octenes can be separated and isolated from the octane diluent by using the process of the present invention with high yield and high selectivity.

### Examples 14-17

When *trans*-2-octene in the presence of trihexylaluminium was subjected to various "Ni" catalysts at 77 °C the isomerisation rate was rather low (26-35 % double bond shift after 2 hours), depending on the character, concentrations and ratios of the aluminium and the nickel compounds.

When, instead of the "Ni" catalysts, the non-isomerising displacement catalyst, Co naphthenate, was used (Example 17) hardly any (< 1 %) double bond isomerisation was observed under further identical conditions.

The experimental details and the results of *trans*-2-octene double bond isomerisation experiments are summarised in Table 2.

**Table 2**

| Example | Ni- or Co- catalyst (Metal catalyst, M) | Metal conc. (ppm) | Al/M (mol/mol) | *trans*-2-octene content after 2 h at 77 °C (%) |
|---|---|---|---|---|
| 14 | Ni(COD)₂ | 29 | 10 | 65 |
| 15 | Ni 2-ethylhexanoate | 35 | 8 | 71 |
| 16 | Ni naphthenate | 11 | 29 | 74 |
| 17 | Co naphthenate | 8 | 38 | 99 |

It is expected that upon reaction of trioctylaluminium with hexadecenes in hexadecane at elevated temperatures the "Ni" catalysed isomerisation/displacement reaction is driven to completion by boiling off the octenes.

It is further expected that if the contact time between the initially formed 1-octene and the "Ni" catalyst in solution is very low, taking into account the observed low rate of "Ni"/Al(hexyl)₃ catalysed isomerisation at 77 °C (Examples 14-17), the octenes may be predominantly isolated as 1-octene in this so-called reactive distillation step (a reaction step where a reaction product is boiled off simultaneously to shift the equilibrium into the desired direction).

## Claims

1. A process for the separation of internal olefins from a mixed feedstock comprising internal olefins and paraffins, wherein said process comprises:
(i) reacting said mixed feedstock with a trialkylaluminium compound in the presence of an isomerisation/displacement catalyst in order to cause isomerisation of the internal olefins and to displace alkyl group(s) from said trialkylaluminium compound to form an alkylaluminium compound wherein at least one of the alkyl groups bound to the aluminium is an alkyl group which has been derived from the isomerisation of said internal olefins;
and
(ii) reacting the alkylaluminium compound formed in step (i) with one or more displacement olefins in the presence of an isomerisation/displacement catalyst in order to cause the displacement olefins to optionally isomerise and to displace alkyl group(s) from the alkylaluminium compound to form a trialkylaluminium compound and to liberate 1-olefins derived from the internal olefins of the mixed feedstock, which 1-olefins are isomerised to form internal olefins.

2. Process according to Claim 1, wherein the mixed feedstock comprises internal olefins and paraffins having in the range of from 6 to 40 carbon atoms.

3. Process according to Claim 1 or 2, wherein the mixed feedstock comprises in the range of from 1 to 40 wt. % of internal olefins, with respect to the total weight of said feedstock.

4. Process according to any one of Claims 1 to 3, wherein the trialkylaluminium compound of step (i) is selected from triethylaluminium, tri-n-propylaluminium, tri-n-butylaluminium, tri-isobutylaluminium, tri-n-hexylaluminium, tri-n-octylaluminium, tri-n-decylaluminium, tri-n-dodecylaluminium, tri-n-tetradecylaluminium, tri-n-hexadecylaluminium, tri-n-octadecylaluminium and mixtures thereof.

5. Process according to any one of Claims 1 to 4, wherein the isomerisation/displacement catalyst in step (i) and/or step (ii) is a nickel catalyst selected from nickel (II) salts, nickel (II) carboxylates, nickel (II) acetonates and nickel (0) complexes and mixtures thereof.

6. Process according to any one of Claims 1 to 5, wherein the displacement olefins used in step (ii) are derived from the displaced alkyl group(s) of the trialkylaluminium compound of step (i).

7. Process according any one of Claims 1 to 6, wherein step (ii) is conducted with a stoichiometric excess of displacement olefins over the amount required to replace all the alkyl group(s) in said alkylaluminium compound.

8. Process according to any one of Claims 1 to 7, wherein the displacement olefins used in step (ii) are selected from internal hexene, internal heptene, internal octene, internal nonene, internal decene, internal undecene, internal dodecene, 1-hexene, 1-octene, 1-decene and 1-dodecene.

9. Process according to any one of Claims 1 to 8, wherein the mixed feedstock is derived from a Fischer-Tropsch process and/or the mixed feedstock is derived from the dehydrogenation of paraffins prior to step (i).

10. Process according to any one of Claims 1 to 9, wherein (a) 1- and internal olefins displaced from the trialkylaluminium compound of step (i) are separated prior to step (ii); and/or (b) the isomerisation/displacement catalyst used in step (ii) and/or residual isomerisation/displacement catalyst remaining from step (i) are deactivated or removed prior to isolating the isomerised internal olefins from the reaction mixture of step (ii).
